# EUROPEAN PATENT APPLICATION

(11) **EP 4 545 643 A1**
(43) Date of publication of application: **30.04.2025**
(21) Application number: 23205325.6
(22) Date of filing: 23.10.2023
(51) Int. Cl.: C12N 15/86

(54) **PLASMIDS FOR IMPROVED AAV TITERS AND EMPTY FULL RATIOS**

(71) Applicant: Sartorius Stedim Cellca GmbH, 89081 Ulm (DE)
(72) Inventor: Jung, Ulrike, 89081 Ulm (DE)
(74) Representative: Cohausz & Florack

(57) **Abstract**

The invention relates to a rep plasmid comprising at least one adeno-associated virus replication protein coding sequence encoding at least one functional rep protein, at least one polyadenylation signal sequence downstream the adeno-associated virus replication protein coding sequence, and a minimal element comprising one or more rep-binding elements downstream the polyadenylation signal sequence In another aspect, the invention provides a transgene plasmid comprising a CMVie promotor, a transgene, SV40 Poly A, a 5' and a 3' inverted terminal repeats derived from J01901.1 AAV2; wherein the transgene plasmid is selected from one of the following: a conventional single-stranded genome recombinant adeno-associated virus, or a self-complementary genome recombinant adeno-associated virus.

## Description

### FIELD OF THE INVENTION

The present invention relates to a rep or rep cap plasmid comprising at least one adeno-associated virus replication protein coding sequence encoding at least one functional rep protein, at least one polyadenylation signal sequence downstream of the adeno-associated virus replication protein coding sequence, and a minimal element comprising one or more rep-binding elements downstream the polyadenylation signal sequence. The invention further concerns a plasmid system for producing an adeno-associated virus particle comprising a transgene plasmid and the rep plasmid of the invention. In another aspect, the invention provides a transgene plasmid comprising a CMVie promotor, a transgene, SV40 PolyA, a 5' and a 3' inverted terminal repeats derived from J01901.1 AAV2; wherein the transgene plasmid is selected from one of the following: a conventional single-stranded genome recombinant adeno-associated virus, or a self-complementary genome recombinant adeno-associated virus. In still another aspect of the invention, a cell comprising the rep plasmid of the invention, the plasmid system of the invention, and/or the transgene plasmid of the invention is provided. In still another aspect of the invention, a kit comprising the expression system or cell is provided. In still another aspect, the invention provides a method of producing recombinant adeno-associated viral vectors comprising transfecting cells with the plasmid system of the invention.

### BACKGROUND OF THE INVENTION

Several gene delivery techniques have been developed to express a gene of interest in cells, tissues, or organisms, including mammalian and in particular human cells, tissues or organisms.

Some of these delivery techniques use a viral vector derived from lentivirus, oncoretrovirus, adenovirus, adeno-associated virus or similar viruses. Among these vectors, the adeno-associated virus (AAV) vector has been recognized as a promising tool. Adeno-associated viruses (AAVs) are linear, single-stranded DNA viruses that belong to the parvovirus family. AAVs are infectious to cells of a wide range of species, including humans, and can infect non-dividing cells in which differentiation has ceased, such as blood cells, muscle or nerve cells. In addition, wild-type AAVs are non-pathogenic to humans, and AAV particles are physiochemically very stable. These characteristics have led to the development of recombinant AAVs (rAAVs) as vectors for gene delivery and in particular gene therapy.

The single-stranded genome of wild-type AAV comprises rep (replication) and cap (capsid) genes. These genes give rise to several rep and cap proteins through alternative translation start sites and differential splicing. The coding sequences are flanked by inverted terminal repeats (ITRs), leading the AAV genome to form a T-shaped hairpin structure through the ITR at both ends, wherein the linear single-stranded genome between the hairpin structures encodes the rep and cap proteins. The rep gene encodes four proteins (rep78, rep68, rep52, and rep40) for viral genome replication and packaging, while cap expression gives rise to the three capsid proteins (VP1, VP2, and VP3), which form the outer capsid shell that protects the AAV genome, as well as being involved in cell binding and internalization.

A recombinant AAV (rAAV), which lacks the wild-type genome, is a protein-based nanoparticle designed to cross the cell membrane where it can transport and deliver its recombinant DNA cargo into the nucleus of a cell. In the absence of the rep proteins, ITR-flanked transgenes encoded by rAAV can form circular concatemers that persist as episomes in the nucleus of transduced cells. Because recombinant episomal DNA does not integrate into the host genome, it will eventually become diluted over time as the cell undergoes repeated rounds of replication. This will eventually lead to loss of the transgene and transgene expression, with the rate of transgene loss depending on the turnover rate of the transduced cell. These characteristics make rAAV ideal for gene therapy applications and AAV-vector-mediated gene delivery was recently approved for the treatment of inherited blindness and spinal muscular atrophy, and long-term therapeutic effects have been achieved for other rare diseases, including hemophilia and Duchenne muscular dystrophy.

Typical recombinant adeno-associated viral vectors (rAAV vectors) have a genome structure in which the rep and cap genes between the ITRs of the wildtype AAV genome are replaced by one or more transgenes to form a transgene plasmid for gene delivery in gene therapy applications. An example of the method of producing an rAAV vector is a method comprising introducing into a host cell a transgene plasmid in which a transgene is inserted between ITRs and introducing a rep plasmid for supplying the rep protein for replication to produce an rAAV vector in the host cell. The rep plasmid can further comprise the cap gene or the cap gene can be located on a separate cap plasmid introduced into the host cell. Furthermore, accessory helper genes from other viruses like Herpes or Adenoviruses can be introduced in the cell to regulate cell metabolism as well as AAV gene expression, prevent apoptosis, and other functions.

While wildtype AAV is capable of providing 100% full and infectious particles, with rAAV the range is usually with 1-30% full (the latter usually achieved rarely and only with very intense effort for particular transgenes and other elements combinations) and only 1 out of 200-1000 full particles infectious. Consequently, when using an rAAV vector for gene therapy applications, it is challenging to produce a sufficiently high titer required for efficient and effective treatment. High levels of product-related impurities, e.g., empty/partially filled viral capsids, during production further challenge rAAV production. Product-related impurities can include AAV empty capsids, encapsidated host cell nucleic acid/helper DNA, and noninfectious AAV capsids. Among these impurities, empty capsids are reported to be the most detrimental to productivity and cause the lot-to-lot variability in products. A purification process that cannot substantially remove this product-related impurity can result in exacerbated immune responses and reduced transduction efficiency. The problem is further exacerbated as during current common purification methods by chromatography the product loss is increasing exponentially with the percentage of empty capsid impurity in the raw bulk product. Accordingly, improving the concentration of viral genomes (i.e., the genomic titer) as well as the full particle (i.e., full capsid) ratio in the raw bulk product is particularly imperative for cost effective gene therapy with AAV and achieving highly pure drug substances for gene therapy applications.

Recent attempts to improve rAAV production include modifying the AAV ITRs so that the transgene is expressed without the need for second-strand DNA synthesis. Further approaches aim to optimize the promoter(s), the AAV transgene(s), the packaging process of the AAV, the AAV capsid to enhance transduction, the AAV vectors to enhance AAV trafficking or uncoating, the plasmids ratio for AAV production, and the subsequent purification process.

### SUMMARY OF THE INVENTION

Against the aforementioned background, it is an object of the present invention to provide improved means for efficient rAAV vector production. In particular it is an object of the present invention to provide rAAV capsids (or rAAV particles) which are properly filled with their intended DNA cargo (referred to as full capsids), while the fraction of empty capsids, meaning of capsids that are not filled with their intended DNA cargo, is reduced. It is thus an object of the invention to provide an improved concentration of rAAV vectors containing the desired DNA. A further object of the invention is to provide an improved concentration of rAAV vectors capable of successfully transducing cells. A further object of the invention is to provide an improved ratio of genome-containing rAAV vectors relative to the total number of viral capsids, which can include empty capsids that are devoid of a genome and the desired gene(s).

These objects are achieved by the rep plasmid of claims 1 to 5, the plasmid system of claims 6 to 9, the transgene plasmid of claim 10 or 11, the stable or transient cell expression system of claim 12, the cell of claim 13, the kit of claim 14, the method of producing recombinant adeno-associated viral vectors of claim 15.

The invention provides a rep plasmid and a plasmid system comprising the rep plasmid for efficient rAAV vector production. This is achieved by providing a minimal element comprising one or more recombinant rep-binding elements downstream the polyadenylation signal sequence. The research underlying this invention has surprisingly found that including at least one recombinant, i.e., non-natural, minimal element comprising the rep-binding element downstream the polyadenylation signal sequence results in improved rAAV vector production. Cells transfected with the rep plasmid of the invention for the production of adeno-associated viral particles provide an increased concentration of viral genomes and full capsids. The rep plasmid of the invention thus is particularly suitable for producing rAAVs for gene therapy applications, wherein a high titer is required for efficient and/or effective treatment.

Further the invention concerns a transgene plasmid, wherein cells transfected with the transgene plasmid of the invention produce an improved concentration of full capsids. This is achieved by the transgene plasmid comprising a CMVie promotor, a transgene, SV40 PolyA, a 5' and a 3' inverted terminal repeats derived from J01901.1 AAV2; wherein the transgene plasmid is selected from one of the following:
(i) conventional single-stranded genome recombinant adeno-associated virus, or
(ii) self-complementary genome recombinant adeno-associated virus.

The transgene plasmid of the invention thus is also particularly suitable for producing rAAVs for gene therapy applications.

The invention further concerns a stable or transient cell expression system comprising the plasmid system of the invention.

The invention further concerns a kit comprising a stable or transient cell expression system of the invention or a cell of the invention, and a cell culture medium. This kit provides improved means for efficient rAAV vector production through the transgene plasmid of the invention and/or rep plasmid of the invention.

The invention further concerns a method for producing rAAV vectors by transfecting cells with the rep plasmid of the invention, the plasmid system of the invention, or the transgene plasmid of the invention.

### DETAILED DESCRIPTION

Although certain embodiments of the present invention are described in detail below, it is to be understood that this invention is not limited to the particular embodiments, methodologies, protocols and reagents described herein as these may vary within the scope set by the claims. It is also to be understood that terminology used herein is for the purpose of describing particular embodiments only, and is not intended to limit the scope of the present invention which is defined by the appended claims. Unless defined otherwise, all technical and scientific terms used herein have the same meanings as commonly understood by one of ordinary skill in the art.

In the following description, certain elements of the present invention will be described. These elements may be discussed with specific embodiments, however, it should be understood that they may be combined in any manner and in any number to create additional embodiments. The variously described examples, features and particular embodiments should not be construed to limit the present invention to only the explicitly described embodiments or to the explicitly described combination of features. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

The above objects are achieved by the following embodiments in accordance with the invention:
1. A rep plasmid comprising:
   (i) at least one adeno-associated virus replication protein coding sequence encoding at least one functional rep protein;
   (ii) at least one polyadenylation signal sequence downstream the adeno-associated virus replication protein coding sequence; and
   (iii) a minimal element comprising one or more recombinant rep-binding elements downstream the polyadenylation signal sequence.
2. The rep plasmid of embodiment 1, wherein the one or more rep-binding elements function as binding site for rep68 and/or rep78.
3. The rep plasmid of embodiment 1 or 2, wherein the minimal element comprises at least two rep-binding elements.
4. The rep plasmid of any of embodiments 1 to 3, comprising a further minimal element comprising one or more rep-binding elements downstream the polyadenylation signal sequence.
5. The rep plasmid of any of embodiments 1 to 4, comprising at least one adeno-associated virus capsid protein coding sequence encoding at least one functional cap protein.
6. The rep plasmid of embodiment 5, wherein the capsid protein coding sequence encodes one or more of VP1, VP2, and VP3, or artificial variants thereof.
7. The rep plasmid of embodiment 5 or 6, wherein the capsid protein coding sequence is derived from AAV2.
8. The rep plasmid of any of embodiments 1 to 7, wherein the rep-binding element comprises the SEQ ID NO: 18 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
9. The rep plasmid of any of embodiments 1 to 8, wherein the rep-binding element is derived from an inverted terminal repeats sequence, preferably derived from a 3'inverted terminal repeats sequence derived from J01901.1 AAV2.
10. The rep plasmid of any of embodiments 1 to 9, wherein the minimal element consists of 500 nt or less, 450 nt or less, 300 nt or less, 250 nt or less, 200 nt or less, preferably 150 nt or less or 100 nt or less.
11. The rep plasmid of any of embodiments 1 to 10, wherein the minimal element comprises the SEQ ID NO: 6 or a sequence having at least 60%, at least 70%, at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
12. The rep plasmid of any of embodiments 1 to 11, wherein the replication protein coding sequence encodes one or more of rep78, rep68, rep52, and rep40, or artificial variants thereof.
13. The rep plasmid of any of embodiments 1 to 12, wherein the replication protein coding sequence is derived from AAV2.
14. The rep plasmid of any of embodiments 1 to 13, wherein the rep plasmid further comprises one or more native or heterologous promoters operably linked to the replication protein coding sequence, preferably wherein the promotors are selected from p5, p19, and p40.
15. A plasmid system for producing an adeno-associated virus particle comprising:
   (i) a transgene plasmid; and
   (ii) the rep plasmid of any one of embodiments 1 to 14.
16. The plasmid system of embodiment 15, wherein the system further comprises a helper plasmid.
17. The plasmid system of embodiment 16, wherein the helper plasmid comprises one or more coding sequences encoding for E2A, E4orf6, or E4orf7 or any combination thereof.
18. The plasmid system of embodiment 16 or 17, wherein the helper plasmid comprises one or more virus-associated RNAs.
19. The plasmid system of any one of embodiments 16 to 18, wherein the transgene plasmid comprises a promotor, a transgene, a polyadenylation signal sequence, 5' and 3' inverted terminal repeats, wherein the transgene plasmid is selected from one of the following:
   (i) conventional single-stranded genome recombinant adeno-associated virus, or
   (ii) self-complementary genome recombinant adeno-associated virus.
20. The plasmid system of embodiment 19, wherein the 3' and 5' inverted terminal repeats are derived from AAV2, preferably J01901.1 AAV2.
21. The plasmid system of embodiment 19 or 20, wherein at least one inverted terminal repeats, preferably the 5' inverted terminal repeats, has a deletion compared to the native inverted terminal repeats.
22. The plasmid system of any one of embodiments 19 to 21, wherein the promoter is a CMVie promoter.
23. The plasmid system of any one of embodiments 15 to 22, wherein the transgene plasmid comprises a transgene that comprises a reporter gene, preferably the transgene is a reporter gene.
24. The plasmid system of embodiment 23, wherein the reporter gene can be detected by antibody-based assays.
25. The plasmid system of embodiment 23 or 24, wherein the reporter gene is a fluorescent molecule.
26. The plasmid system of embodiment 22 or 24, wherein the reporter gene is a beta-galactosidase, luciferase or glutathione S-transferase.
27. The plasmid system of any one of embodiments 19 to 26, wherein the polyadenylation signal sequence is a SV40 poly(A) signal sequence.
28. The plasmid system of any one of embodiments 15 to 27, wherein the transgene plasmid comprises the following structure:
   5'ITR(flip)-CMVie promotor-transgene-SV40 PolyA-ITR(flop)3'.
29. The plasmid system of embodiment 28, wherein the inverted terminal repeats with the flip orientation has the SEQ ID NO: 1 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
30. The plasmid system of embodiment 28 or 29, wherein the inverted terminal repeats with the flop orientation has the SEQ ID NO: 2 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
31. The plasmid system of any one of embodiments 15 to 30, wherein the transgene plasmid does not contain a woodchuck hepatitis posttranscriptional regulatory element.
32. The plasmid system of any one of embodiments 15 to 31, further comprising a cap plasmid.
33. The plasmid system of embodiment 32, wherein the cap plasmid comprises at least one adeno-associated virus capsid protein coding sequence encoding at least one functional cap protein.
34. The plasmid system of embodiment 33, wherein the capsid protein coding sequence encodes one or more of VP1, VP2, and VP3, or artificial variants thereof.
35. The plasmid system of any one of embodiments 33 or 34, wherein the capsid protein coding sequence is derived from AAV2.
36. A transgene plasmid comprising:
   a CMVie promotor, a transgene, SV40 Poly A, a 5' and a 3' inverted terminal repeats derived from J01901.1 AAV2;
   wherein the transgene plasmid is selected from one of the following:
      (i) conventional single-stranded genome recombinant adeno-associated virus, or
      (ii) self-complementary genome recombinant adeno-associated virus.
37. The transgene plasmid of embodiment 36, wherein at least one inverted terminal repeats, preferably the 5' inverted terminal repeats, has a deletion compared to the native inverted terminal repeats, further preferably wherein the inverted terminal repeats comprising the deletion has the SEQ ID NO: 4 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
38. The transgene plasmid of embodiment 36 or 37, wherein the transgene plasmid comprises the following structure:
   5'ITR(flip)-CMVie promotor-transgene-SV40 PolyA-ITR(flop)3'.
39. The transgene plasmid of embodiment 38, wherein the inverted terminal repeats with the flip orientation has the SEQ ID NO: 1 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO
40. The transgene plasmid of embodiment 38 or 39, wherein the inverted terminal repeats with the flop orientation has the SEQ ID NO: 2 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO
41. The transgene plasmid of any one of embodiments 36 to 40, wherein the transgene is a reporter gene, preferably the reporter gene can be detected by antibody-based assays, more preferably the reporter gene is a fluorescent molecule or the reporter gene is a beta-galactosidase, luciferase or glutathione S-transferase.
42. The transgene plasmid of any one of embodiments 36 to 41, wherein the plasmid does not contain a woodchuck hepatitis posttranscriptional regulatory element.
43. A rep-cap plasmid comprising:
   (i) at least one adeno-associated virus replication protein coding sequence encoding at least one functional rep protein;
   (ii) at least one adeno-associated virus capsid protein coding sequence encoding at least one functional cap protein;
   (iii) at least one polyadenylation signal sequence downstream the adeno-associated virus replication protein coding sequence; and
   (iv) a minimal element comprising one or more recombinant rep-binding elements downstream the polyadenylation signal sequence.
44. The rep-cap plasmid of embodiment 43, wherein the one or more rep-binding elements function as binding site for rep68 and/or rep78.
45. The rep-cap plasmid of embodiment 43 or 44, wherein the minimal element comprises at least two rep-binding elements.
46. The rep-cap plasmid of any of embodiments 43 to 45, comprising a further minimal element comprising one or more rep-binding elements downstream the polyadenylation signal sequence.
47. The rep-cap plasmid of any of embodiments 43 to 46, wherein the capsid protein coding sequence encodes one or more of VP1, VP2, and VP3, or artificial variants thereof.
48. The rep-cap plasmid of any of embodiments 43 to 47, wherein the capsid protein coding sequence is derived from AAV2.
49. The rep-cap plasmid of any of embodiments 43 to 48, wherein the rep-binding element comprises the SEQ ID NO: 18 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
50. The rep-cap plasmid of any of embodiments 43 to 49, wherein the rep-binding element is derived from an inverted terminal repeats sequence, preferably derived from a 3'inverted terminal repeats sequence derived from J01901.1 AAV2.
51. The rep-cap plasmid of any of embodiments 43 to 50, wherein the minimal element consists of 500 nt or less, 450 nt or less, 300 nt or less, 250 nt or less, 200 nt or less, preferably 150 nt or less or 100 nt or less.
52. The rep-cap plasmid of any of embodiments 43 to 51, wherein the minimal element comprises the SEQ ID NO: 6 or a sequence having at least 60%, at least 70%, at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.
53. The rep-cap plasmid of any of embodiments 43 to 52, wherein the replication protein coding sequence encodes one or more of rep78, rep68, rep52, and rep40, or artificial variants thereof.
54. The rep-cap plasmid of any of embodiments 43 to 53, wherein the replication protein coding sequence is derived from AAV2.
55. The rep-cap plasmid of any of embodiments 43 to 54, wherein the rep plasmid further comprises one or more native or heterologous promoters operably linked to the replication protein coding sequence, preferably wherein the promotors are selected from p5, p19, and p40.
56. A stable or transient cell expression system comprising the plasmid system of any one of embodiments 15 to 35 and a cell line.
57. A cell comprising the rep plasmid of any of embodiments 1 to 14, the plasmid system of any one of embodiments 15 to 35, the transgene plasmid of any one of embodiments 36 to 42, or the rep-cap plasmid of any one of embodiments 43 to 55.
58. The cell of embodiment 57, wherein the cell is a mammalian cell.
59. A kit comprising a stable or or transient cell expression system of embodiment 56, or a cell of any one of embodiment 57 or 58, and a cell culture medium.
60. The kit of embodiment 59, further comprising media feeds, media additives or transfection reagents or any combination thereof.
61. The kit of embodiment 59 or 60, further comprising a manual.
62. A method of producing recombinant adeno-associated viral vectors comprising:
   (i) transfecting cells with the rep plasmid of any of embodiments 1 to 14, the plasmid system of any one of embodiments 15 to 35, the transgene plasmid of any one of embodiments 360 to 42, or the rep-cap plasmid of any one of embodiments 43 to 55;
   (ii) culturing the transfected cells to produce said adeno-associated viral vectors; and
   (iii) isolating said recombinant adeno-associated viral vectors.
63. The method of producing recombinant adeno-associated viral vectors of embodiment 62, wherein the cells are HEK293 cells.

### Definitions

The terms indicated for explanation of the invention have the following meaning, unless otherwise indicated in the description or the claims. Additional definitions are set forth throughout the detailed description.

The "3'UTR sequence" is a 3' untranslated region known to regulate mRNA-based processes, such as mRNA localization, mRNA stability, and translation. In addition, 3' UTRs can establish 3' UTR-mediated protein-protein interactions (PPIs), and thus can transmit genetic information encoded in 3' UTRs to proteins. This function has been shown to regulate diverse protein features, including protein complex formation or posttranslational modifications, but is also expected to alter protein conformations.

The "5' UTR sequence" is a 5'-untranslated region which lies within the noncoding genome upstream of a coding sequence and plays an important role in regulating gene expression. Within 5'-UTR sequences may be numerous cis-regulatory elements present that can interact with the transcriptional machinery to regulate mRNA abundance. The 5'-untranslated region may contain various RNA-based regulatory elements including the secondary structures, RNA-binding protein motifs, upstream open-reading frames (uORFs), internal ribosome entry sites, terminal oligo pyrimidine (TOP) tracts, and G-quadruplexes. These elements can alter the efficiency of mRNA translation; some can also affect mRNA transcript levels via changes in stability or degradation.

Terms "a" and "an" and "the" and similar reference used in the context of describing the invention (especially in the context of the claims) are to be construed to cover both the singular and the plural, unless otherwise indicated herein or clearly contradicted by context.

The terms "about" or "approximately" as used herein denotes a range of ±10% of a reference value. For examples, "about 10" defines a range of 9 to 11. In general, those skilled in the art, familiar with the context, will appreciate the relevant degree of variance encompassed by "about" or "approximately" in that context.

As used herein, the term "adeno-associated virus" (AAV), includes but is not limited to, AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

Also as used herein, "and/or" refers to and encompasses any and all possible combinations of one or more of the associated listed items, as well as the lack of combinations when interpreted in the alternative ("or"). The use of the alternative (e.g., "or") should be understood to mean either one, both, or any combination thereof of the alternatives.

A "cap plasmid" is a plasmid comprising a capsid protein coding sequence. The terms "cap plasmid" and "capsid plasmid" can be used interchangeably herein.

Unless expressly specified otherwise, the term "comprising" is used in the context of the present document to indicate that further members may optionally be present in addition to the members of the list introduced by "comprising". It is, however, contemplated as specific embodiments of the present invention that each time the term "comprising" is used, this shall also encompass the possibility of no further members being present, i.e., for the purpose of this embodiment "comprising" can be understood as having the meaning of "consisting of".

As used herein "derived from" in the context of a nucleic acid or amino acid sequence means that the sequence is identical to a sequence from which it is derived or has a specified percentage of amino acid residues or nucleotides that are the same as the sequence from which it is derived. The specified percentage can be about at least 60%, preferably at least 65%, at least 70%, at least 75%, at least 80%, at least 85%, at least 90%, at least 91%, at least 92%, at least 93%, at least 94%, at least 95%, at least 96%, at least 97%, at least 98%, at least 99% over a specified sequence, when compared and aligned for maximum correspondence over a comparison window or designated region as measured using a BLAST or BLAST 2.0 sequence comparison algorithms with default parameters, or by manual alignment and visual inspection. For example, the 3' and 5' inverted terminal repeats can be derived from AAV2, preferably J01901.1 AAV2, meaning, the 3' and 5' inverted terminal repeats can be identical to the 3' and 5' inverted terminal repeats comprised in the AAV2 genome, preferably J01901.1 AAV2 genome, or can be at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% identical to the 3' and 5' inverted terminal repeats comprised in the AAV2 genome, preferably J01901.1 AAV2 genome.

Herein, the term "DNA" relates to a nucleic acid molecule which is entirely or at least substantially composed of deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof). DNA may be recombinant DNA and may be obtained by cloning of a nucleic acid, in particular cDNA. The cDNA may be obtained by reverse transcription of RNA.

A "feed" or "supplement" as used herein refers to a composition when added to cells in standard culture may be beneficial for its maintenance, or expansion, or growth, or viability, or affects its cell performance, or increases culture longevity or maintaining cells in a pseudo-stationary phase wherein product expression continues, or results in an increase in final product titer. A feed or supplement may be used interchangeably in this disclosure and refers to solid and liquid formats (including agglomerated formats) of media components comprising one or more amino acids, sugars, vitamins, buffers, sometimes, peptides, hydrolysates, fractions, growth factors, hormones, etc. required to rebalance or replenish or to modulate the growth or performance of a cell in culture, or a cell culture system. A feed or supplement may be distinguished from a cell culture medium in that it is added to a cell culture medium that can culture a cell. As would be understood by one of skill in the art, sometimes a feed/supplement may comprise mainly those amino acids, sugars, vitamins, buffers, etc. required to rebalance or replenish or modulate the growth or performance of a cell in culture, or a cell culture system. A feed or supplement may or may not be concentrated or may be partially concentrated for certain components only.

As used herein, the term "gene" refers to the segment of a DNA molecule that codes for a polypeptide chain (e.g., the coding region). In some embodiments, a gene is positioned by regions immediately preceding, following, and/or intervening the coding region that are involved in producing the polypeptide chain (e.g., regulatory elements such as a promoter, enhancer, polyadenylation sequence, 5'-untranslated region, 3'-untranslated region, or intron).

As used herein, the term "encode" or "encoding" refers to sequence information of a first molecule that guides production of a second molecule having a defined sequence of nucleotides (e.g., mRNA) or a defined sequence of amino acids. For example, a DNA molecule can encode an RNA molecule (e.g., by a transcription process that includes a DNA-dependent RNA polymerase enzyme). A coding sequence encoding a protein is a sequence that guides production of said protein. Thus, a cording sequence, a gene, a cDNA, or a single-stranded RNA (e.g., an mRNA) encodes a polypeptide if transcription and translation of mRNA corresponding to that gene produces the polypeptide in a cell or other biological system. In some embodiments, a coding sequence encoding a target polypeptide refers to a coding strand, the nucleotide sequence of which can be identical to the mRNA sequence of such a target polypeptide. In some embodiments, a coding sequence encoding a target polypeptide refers to a non-coding strand of such a target polypeptide agent, which may be used as a template for transcription of a gene or cDNA. As is understood in the art, the phrase "coding sequence encoding a peptide or protein" means that the plasmid containing the coding sequence, if present in the appropriate environment, for example within a cell and/or in a cell-free translation system, can direct the assembly of amino acids to produce the peptide or protein via a process of translation.

A functional rep protein in context of the invention is a replication (rep) protein involved in AAV replication and/or AAV assembly. In some embodiments, the functional rep protein is involved in AAV replication and viral assembly. In some embodiments, the functional rep protein is involved in AAV replication. In some embodiments, the functional rep protein is involved in AAV virion assembly. In some embodiments, the functional rep protein binds to DNA. In some embodiments, the functional rep protein does not bind to DNA.

A functional cap protein in context of the invention is a cap protein capable of forming a capsid. A functional cap protein can be any of VP1, VP2, VP3 or artificial variants thereof capable of forming a capsid.

The terms "identical" or percent (%) "identity," in the context of two or more nucleic acids or peptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection.

Inverted terminal repeats (ITRs) are guanine-cytosine-rich structures involved in the replication and encapsidation of the AAV genome, along with its integration in and excision from the host genome. ITRs are natural AAV-derived DNA sequences conserved in recombinant AAV (rAAV), as they allow its replication, encapsidation, and long-term maintenance and expression in target cells. ITRs can be incomplete, truncated, and/or modified. An ITR can be a truncated, meaning one or more nucleotides of the natural sequence can be deleted. An ITR can be modified, meaning an ITR can comprise one or more nucleotides of non-ITR sequences. The non-ITR sequence can be inserted within the ITR sequence or the non-ITR sequence can be added at one or both ends of the ITR sequence. The modified ITR sequence can comprise substitution wherein one or more nucleotides are exchanged. It has been shown that rAAV genomes can be replicated, even with incomplete, truncated, or modified ITR sequences. It is understood that the present invention is not limited to a specific ITR sequence and any ITR sequence now known or later discovered can be used.

A "minimal element" is a recombinant polynucleotide sequence comprising one or more rep-binding elements.. The minimal element is preferably a recombinant minimal element. The minimal element is preferably a short polynucleotide sequence, preferably having a length of 500 nt or less, more preferably 500 nt or less, more preferably 450 nt or less, more preferably 400 nt or less, more preferably 350 nt or less, more preferably 300 nt or less, more preferably 250 nt or less, more preferably 200 nt or less, most preferably 150 nt or less or 100 nt or less. The minimal element can comprise a nucleotide sequence which is not naturally occurring in AAV. This non-natural nucleotide sequence can be upstream and/or downstream the one or more rep-binding elements. In some embodiments, the minimal element comprises one rep-binding element, preferably comprises one rep-binding element and non-natural nucleic acids. In some embodiments the minimal element comprises or consists of two or more rep-binding elements, preferably comprises two or more rep-binding element. In some embodiments, the minimal element is derived from an ITR sequence. In some embodiments, the minimal element is derived from a truncated and/or modified ITR sequence.

As used herein, a nucleic acid sequence (e.g., coding sequence) and regulatory sequences are said to be "operatively linked" when they are covalently linked in such a way as to place the expression or transcription of the nucleic acid sequence under the influence or control of the regulatory sequences. If it is desired that the nucleic acid sequences be translated into a functional protein, two DNA sequences are said to be operatively linked if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein.

A "polyadenylation signal sequence" (PAS) is a sequence known to those skilled in the art. The polyadenylation signal sequence typically comprises a conserved hexamer motif required for the polyadenylation of an mRNA, a U rich and/or GU rich sequence downstream of the hexamer motif, and a dinucleotide sequence that precedes the cleavage site for polyadenlation and is located between the U rich and/or GU rich sequence and the hexamer motif. The sequence can be recognised by cleavage and polyadenylation specificity factor (CPSF) within an RNA cleavage complex. The hexamer motif varies between eukaryotes but can be AATAAA or modifications thereof. CPSF is the central component of the 3' processing machinery for polyadenylated mRNAs and recognizes the PAS, thereby providing sequence specificity in both pre-mRNA cleavage and polyadenylation, and catalyzes pre-mRNA cleavage. In some embodiments, the polyadenylation signal sequence can be a sequence comprising AATAAA or a modified sequence thereof. The modified sequence of AATAAA can be a sequence in which one or two nucleic acids are deleted, substituted, inserted and/or added. Other PAS are known, for example, ATTAAA, AGTAAA, TATAAA, CATAAA, GATAAA, AATATA, AATACA, AATAGA, AAAAAG, and ACTAAA, and can be used in context of the present disclosure.

A "poly(A)-tail" (or "poly(A)-sequence") is a adenine nucleotide chain typically added to a mRNA molecule during RNA processing to increase the stability of the molecule and enable translation. This process, called polyadenylation, usually adds 100 to 250 adenines.

The terms "nucleic acid sequence" "nucleotide sequence", "polynucleotide" and "nucleic acid" can be used interchangeably herein to refer to one or more nucleotides, preferably deoxyribonucleic acid (DNA). These terms comprise DNA, ribonucleic acid (RNA), combinations thereof, and modified forms thereof. The term comprises genomic DNA, cDNA, mRNA, recombinantly produced and chemically synthesized molecules. In some preferred embodiments, a polynucleotide is DNA. In some embodiments, a polynucleotide is a mixture of DNA and RNA. A polynucleotide may be present as a single-stranded or double-stranded and linear or covalently circularly closed molecule. A polynucleotide can be isolated. The term "isolated polynucleotide " means, according to the present disclosure, that the polynucleotide (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "plasmid" refers to an extrachromosomal circular DNA capable of being expressed in a given cell. Plasmids can also be engineered by standard molecular biology techniques (Sambrook et al., Laboratory Manual, Cold Spring Harbor Laboratory Press, Cold Spring Harbor (1989), N.Y.).

The terms "polypeptide," "peptide," and "protein" are used interchangeably herein to refer to polymers of amino acids.

The term "recombinant" when used in the context of a polynucleotide is a polynucleotide having nucleotide sequences that are not naturally joined together and can be made by artificially combining two otherwise separated segments of sequence. This artificial combination is often accomplished by chemical synthesis or, more commonly, by the artificial manipulation of isolated segments of nucleic acids, for example, by genetic engineering techniques. Recombinant polynucleotides include vectors comprising an amplified or assembled polynucleotide, which can be used to transform or transfect a suitable host cell. A host cell that comprises the recombinant polynucleotide is a "recombinant host cell." The polynucleotide is then expressed in the recombinant host cell to produce a "recombinant polypeptide." A recombinant polynucleotide can also comprise a non-coding function.

A "rep plasmid" is a plasmid comprising a replication protein coding sequence. The terms "rep plasmid" and "replication plasmid" can be used interchangeably herein.

A "rep cap plasmid" or "rep-cap plasmid" is a plasmid comprising a replication protein coding sequence and a capsid protein coding sequence. The terms "rep cap plasmid" and "replication capsid plasmid" can be used interchangeably herein.

"Recombinant AAV" (rAAV) and "AAV" are used interchangeably throughout the application.

As used herein, a "transgene" is a nucleic acid that is introduced into the cell, including but not limited to genes or nucleic acid having sequences which are not normally present in AAV, genes which are present but not normally transcribed and translated ("expressed") in an AAV genome, or any other gene or nucleic acid which one desires to position between the ITR sequences. A transgene may include one or more transcriptional regulatory sequences and any other nucleic acid, such as introns, that may be necessary for optimal expression of a selected nucleic acid. A transgene can be as few as a couple of nucleotides long, but can preferably be at least about 50, 100, 150, 200, 250, 300, 350, 400, or 500 nucleotides (nt) long. A transgene can comprise coding or non-coding sequences.

As used herein, the term "variant" refers to a molecule, such as a gene or protein, that shares one or more particular structural features, elements, components, or moieties with a reference molecule. A variant therefore is a molecule, such as a gene or protein, that shares one or more particular structural features, elements, components, or moieties with a reference molecule. A variant can be a gene that shares one or more particular structural features, elements, components, or moieties with a reference gene. A variant can be a protein that shares one or more particular structural features, elements, components, or moieties with a reference protein. Typically, a "variant" has significant structural similarity with the reference molecule, for example sharing a core or consensus structure, but also differs in certain discrete ways. In some embodiments, a variant is a molecule that can be generated from the reference molecule, e.g., by chemical manipulation of the reference molecule. In some embodiments, a variant is a molecule that can be generated through performance of a synthetic process substantially similar to (e.g., sharing a plurality of steps with) one that generates the reference molecule. In some embodiments, a variant is or can be generated through performance of a synthetic process different from that used to generate the reference molecule.

As used herein, the terms "virus vector," "viral vector," and "gene delivery vector" refer to a virus particle that functions as a nucleic acid delivery vehicle, and which comprises a nucleic acid molecule packaged within a viral capsid. Exemplary virus vectors include adeno-associated virus vectors (AAVs).

Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

References to "one embodiment," "an embodiment," "example embodiment," "some embodiments," "certain embodiments," "various embodiments," etc., indicate that the embodiment(s) of the disclosed technology so described may include a particular feature, structure, or characteristic, but not every embodiment necessarily includes the particular feature, structure, or characteristic.

All patents, patent applications, and other publications cited in this application are incorporated by reference in the entirety for all purposes.

### The rep plasmid of the invention

The present invention provides a rep plasmid comprising:
(i) at least one adeno-associated virus replication protein coding sequence encoding at least one functional rep protein;
(ii) at least one polyadenylation signal sequence downstream the adeno-associated virus replication protein coding sequence; and
(iii) a minimal element comprising one or more recombinant rep-binding elements downstream the polyadenylation signal sequence.

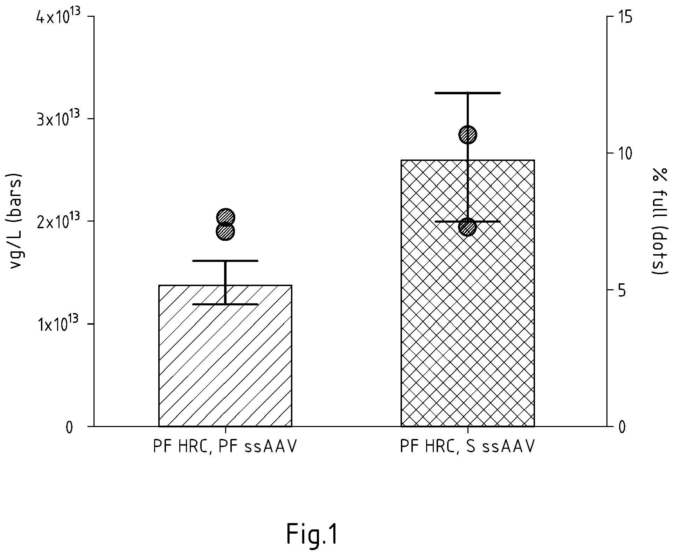

Without wishing to be bound by theory, adding a recombinant rep-binding element downstream the polyadenylation signal sequence is contemplated to improve replication and/or rAAV assembly. As demonstrated in the examples (see Fig. 1-6), the recombinant rep-binding element downstream the polyadenylation signal sequence leads to an improved concentration of viral genomes and full capsids, and thus leads to an improved titer. For this reason, the rep plasmid of the invention is particularly suitable for rAAV production used for gene therapy applications, resulting in fewer empty capsids impurities, which are reported to be the most detrimental to productivity and cause the lot-to-lot variability in products. In addition, due to the high titer an improved transduction efficiency can be obtained.

It has been further surprisingly found that the combination of the rep plasmid of the invention with a self-complementary AAV (scAAV) transgene plasmid provides a synergistic effect regarding the concentration of viral genomes and full capsids.

In an alternative aspect, the recombinant rep-binding element downstream is located between the stop codon of the replication protein coding sequence and the polyadenylation signal sequence, meaning the recombinant rep-binding element downstream is located between downstream the stop codon of the replication protein coding sequence and upstream the polyadenylation signal sequence. In a further aspect, the recombinant rep-binding element downstream is located between the stop codon of a capsid protein coding sequence and the polyadenylation signal sequence, meaning the recombinant rep-binding element downstream is located between downstream the stop codon of the capsid protein coding sequence and upstream the polyadenylation signal sequence.

In some embodiments, the rep plasmid is a rep-cap plasmid further comprising at least one adeno-associated virus capsid protein coding sequence encoding at least one functional cap protein.

Analytical techniques capable of providing information regarding the concentration of viral genomes and full capsids are well-known. Exemplary methods for the detection of the capsid titer of an adeno-associated virus (AAV) and method for the determination of the ratio of full and empty capsids of an AAV are described in WO 2023/139224 incorporated by reference in its entirety. For example, the percentage of genome-containing viral capsids is typically quantified by electron microscopy of a viral vector solution. Size exclusion chromatography coupled to multiangle light scattering (SEC-MALS) can be used to characterize capsid content in addition to other quality attributes. Density-based separation by analytical ultracentrifugation (AUC) can also be employed to resolve empty, intermediate, and full capsids and to quantitate the levels of each population. Charge-based chromatographic separation based on differences in empty and full capsids can also be used to quantitate an empty-to-full capsid ratio. Preferred methods for the determination and quantification of the genome titer are quantitative polymerase chain reaction (qPCR) and digital droplet PCR (ddPCR), quantify the DNA content via fluorescence during or after amplification in a thermocycler. Preferred method for capsid quantification is a conventional enzyme-linked immunosorbent assay (ELISA).

The minimal element is preferably a recombinant minimal element. The minimal element can be a recombinant polynucleotide sequence comprising one or more rep-binding elements downstream the polyadenylation signal sequence. In some embodiments, the minimal element is a polynucleotide sequence which comprises one or more rep-binding elements downstream the polyadenylation signal sequence.

In some embodiments, the minimal element is a short polynucleotide sequence, preferably having a length of 500 nt or less, more preferably 500 nt or less, more preferably 450 nt or less, more preferably 400 nt or less, more preferably 350 nt or less, more preferably 300 nt or less, more preferably 250 nt or less, more preferably 200 nt or less, most preferably 150 nt or less or 100 nt or less.

In some embodiments, the minimal element comprises or consists of one or more rep-binding elements. In some embodiments, the minimal element has a length of preferably 5-500 nt, preferably 10-400 nt, preferably 20-300 nt, preferably 30-200 nt, preferably 40-150 nt. In other embodiments, the minimal element has a length of preferably 5-300 nt, preferably 10-250 nt, preferably 20-200 nt, or preferably 20-150 nt. In some embodiments, the minimal element has a length corresponding to the length of the rep-binding element. In other embodiments, the minimal element has a length corresponding to the length of the rep-binding element and further nucleic acids, which are not considered as rep-binding element nucleic acids, which may be termed non-natural nucleotide sequence as disclosed herein.

In some particularly preferred embodiments, the minimal element is in a reverse orientation.

In some preferred embodiments, the minimal element is derived from an ITR sequence, preferably a 3'ITR sequence, more preferably a 3'ITR sequence from AAV2, even more preferably a 3' ITR from J01901.1 AAV2. As demonstrated in the examples (see Fig. 1-5), the minimal element leads unexpectedly to a significantly higher titer and full capsid concentration during AAV production. This is particularly advantageous when used for gene therapy, when high concentrations of AAV are required for treatment. Furthermore, this simplified the AAV production process, reducing the need for purifications processes that reduced the AAV yield.

In some preferred embodiments, the minimal element is derived from a truncated and/or modified ITR sequence, preferably a truncated and/or modified 3'ITR sequence, more preferably a truncated and/or modified 3'ITR sequence from AAV2, even more preferably a truncated and/or modified 3' ITR from J01901.1 AAV2. The minimal element can comprise the rep-binding element derived from an ITR sequence, preferably 3'ITR sequence, and a non-natural nucleotide sequence. In this context it is understood that the non-natural sequence may not be derived from a sequence immediately adjacent to the rep-binding element sequence of the ITR. For example, the non-natural sequence can be a sequence located further upstream or downstream of the rep-binding element in the ITR.

In some embodiments, the minimal element is derived from an ITR sequence of any AAV serotype, including but not limited to, AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

In some embodiments, the minimal element allows one or more rep proteins to bind to the polynucleotide sequence. The minimal element preferably can allow initiating genomic replication. The replicated sequence can further initiate subsequent rounds of genomic replication.

The minimal element can comprise a nucleotide sequence which is not naturally occurring in AAV or the sequence from which it is derived. The non-natural nucleotide sequence can be upstream and/or downstream the rep-binding element. In some embodiments, the non-natural nucleotide sequence can be upstream, downstream, and/or within the rep-binding element. The non-natural nucleotide sequence can be upstream and/or downstream the rep-binding elements. In some embodiments, the non-natural nucleotide sequence can be upstream, downstream, between and/or within the rep-binding elements.

In some embodiments, the minimal element comprises one rep-binding element. In some embodiments the minimal element comprises two or more rep-binding elements. In some embodiments, the minimal element consists of one rep-binding element. In some embodiments the minimal element consists of two or more rep-binding elements. If the minimal element comprises two or more rep-binding elements, these rep-binding elements can be derived from different AAV serotypes. For example, one rep-binding element can be derived from AAV2, while one rep-binding element can be derived from AAV3.

In some embodiments, the minimal element is derived from a non-ITR sequence, such as an AAV prompter sequence. In some embodiments, the rep-binding element is derived from a p5 promoter. However, in preferred embodiments, the minimal element is not derived from a p5 promoter. In preferred embodiments, the one or more rep-binding element is not derived from a p5 promoter.

In some preferred embodiments, the one or more rep-binding elements function as binding site for rep68 and/or rep78. In some preferred embodiments, the one or more rep-binding elements function as binding site for rep68 or rep78. In some embodiments, the one or more rep-binding elements function as binding site for rep68 and rep78. In some embodiments, the one or more rep-binding elements function as binding site for rep68. In some embodiments, the one or more rep-binding elements function as binding site for rep78.

In some embodiments, the minimal element comprises at least two rep-binding elements. In some embodiments, the minimal element comprises at least three rep-binding elements. In some embodiments, the minimal element comprises at least four rep-binding elements. In some embodiments, the minimal element comprises at least five rep-binding elements. In some embodiments, the minimal element comprises at least six rep-binding elements. In some embodiments, the minimal element comprises at most two rep-binding elements. In some embodiments, the minimal element comprises at most three rep-binding elements. In some embodiments, the minimal element comprises at most four rep-binding elements. In some embodiments, the minimal element comprises at most five rep-binding elements. In some embodiments, the minimal element comprises at most six rep-binding elements.

In some embodiments, the rep plasmid comprises an additional minimal element comprising one or more rep-binding elements. The additional minimal element can be located downstream or upstream of the polyadenylation signal.

In some embodiments, the rep plasmid comprises at least one adeno-associated virus capsid protein coding sequence encoding at least one functional cap protein. In some embodiments, the capsid protein coding sequence encodes one or more of VP1, VP2, and VP3, or artificial variants thereof. In some embodiments, the rep plasmid does not comprise a capsid protein coding sequence. In such embodiments, the rep plasmid may therefore not comprise a p40 promoter as this promoter is typically used for cap transcription.

In preferred embodiments, the VP1 protein comprises or consists of the SEQ ID NO: 12 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the VP2 protein comprises or consists of the SEQ ID NO: 13 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the VP3 protein comprises or consists of the SEQ ID NO: 14 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In a further preferred embodiment, capsid protein (cap) coding sequence is derived from AAV2. In some embodiments, the capsid protein coding sequence is derived from an AAV serotype selected from the group consisting of AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

In some embodiments, rep and/or cap coding sequence may be derived from AAV2, AAV9, and AAV5 serotypes.

In preferred embodiments, the rep-binding element comprises the SEQ ID NO: 18 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO. In preferred embodiments, the rep-binding element consists of the SEQ ID NO: 18 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the rep-binding element is derived from an inverted terminal repeats sequence, preferably derived from a 3'inverted terminal repeats sequence derived from J01901.1 AAV2.

In preferred embodiments, the replication protein (rep) coding sequence is derived from AAV2. In some embodiments, the replication protein coding sequence is derived from an AAV serotype selected from the group consisting of AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered.

In preferred embodiments, the minimal element has a sequence length of 500 nt or less, 450 nt or less, 300 nt or less, 250 nt or less, 200 nt or less, preferably 150 nt or less or 100 nt or less.

In preferred embodiments, the minimal element comprises the SEQ ID NO: 6 or a sequence having at least 60%, at least 70%, at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO. In preferred embodiments, the minimal element consists of the SEQ ID NO: 6 or a sequence having at least 60%, at least 70%, at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the replication protein coding sequence encodes one or more of rep78, rep68, rep52, and rep40, or artificial variants thereof. The coding sequence of the rep proteins, rep78, rep68, rep52, and/or rep40, can be situated in the same open reading frame. The rep proteins generally are translated from transcripts generated by the p5 and p 19 promoter.

In preferred embodiments, the rep78 protein comprises or consists of the SEQ ID NO: 8 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the rep68 protein comprises or consists of the SEQ ID NO: 9 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the rep52 protein comprises or consists of the SEQ ID NO: 10 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the rep40 protein comprises or consists of the SEQ ID NO: 11 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the rep plasmid further comprises one or more native or heterologous promoters operably linked to the replication protein coding sequence, preferably wherein the promotors are selected from p5, p19, and p40.

In some embodiments, the rep plasmid can comprise an AAV promoter selected from p5, p19, and/or p40. The promoter can be derived from a different serotype than the replication protein coding sequence. The promoter can be derived from a different serotype than the capsid protein coding sequence. In some embodiments, the rep plasmid can comprise a non-natural promoter, meaning a promoter which is not derived from AAV. In some embodiments, the rep plasmid comprises a mouse mammary tumor virus (MMTV) promoter. In some embodiments, a MMTV promoter is operatively linked to the replication protein coding sequence. In some embodiments, a MMTV promoter is operatively linked to the capsid protein coding sequence

In some embodiments, the replication protein coding sequence can encode for one or more rep protein hybrids from different AAV serotypes. In preferred embodiments, the replication protein coding sequence is derived from AAV2. In preferred embodiments, the replication protein coding sequence is derived from AAV2 and the promoter operatively linked to the replication protein coding sequence is a MMTV promoter.

In some embodiments, the rep plasmid may include helper genes. These helper genes can be selected from Ela, Elb, E2A, E4orf6, E4orf7, or VA RNA or any combination thereof.

In some embodiments, the rep plasmid is used for rAAV production, wherein the rAAV can be used in gene therapy applications. In some embodiments, the rAAV production is a transient production.

The rep plasmid can comprise one or more 5' and/or 3' UTRs upstream and/or downstream the replication protein coding sequence. In some embodiments, the rep plasmid further comprises regulatory sequences including promoter, binding sites, and/or non-coding RNA. These sequences can be independently derived from AAV, including different AAV serotypes.

In preferred embodiments, the rep plasmid comprises the SEQ ID NO: 7 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO. In preferred embodiments, the rep plasmid consists of the SEQ ID NO: 7 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

### The plasmid system of the invention

The present invention provides a plasmid system for producing an adeno-associated virus particle comprising:
(i) a transgene plasmid; and
(ii) the rep plasmid of the invention.

As shown in the figures 1-6, the plasmid system of the invention surprisingly leads to an improved concentration of viral genomes and full capsids due to the rep plasmid comprising the minimal element comprising a recombinant rep-binding element downstream the polyadenylation signal sequence.

In preferred embodiments, the plasmid system further comprises a helper plasmid. Typically, AAV can be replication defective and may require co-infection by adenovirus or herpes virus in order to replicate efficiently. The individual adenoviral genes that contribute to AAV helper function are well-known and have been inter alia identified by testing adenovirus mutants for their ability to mediate AAV replication. In a further preferred embodiment, the helper plasmid comprises one or more coding sequences encoding for Ela, Elb, E2A, E4orf6, E4orf7 or VA RNA or any combination thereof. These genes (i.e., helper genes) are known to support or participate in AAV replication.

In preferred embodiments, the helper plasmid comprises one or more virus-associated RNAs. Adenovirus 'virus-associated' RNAs (VA RNAs) are suggested to be abundant, heterogeneous, non-coding RNA transcripts, typically comprising 150-200 nucleotides. For instance, VA RNAI is recognized for its function in relieving the cellular anti-viral blockade of protein synthesis through inhibition of the double-stranded RNA-activated protein kinase (PKR). More recent evidence has revealed that VA RNAs interfere with several other host cell processes.

In preferred embodiments, the transgene plasmid comprises a promotor, a transgene, a polyadenylation signal sequence, 5' and 3' inverted terminal repeats, wherein the transgene plasmid is selected from one of the following:
(i) conventional single-stranded genome recombinant adeno-associated virus, or
(ii) self-complementary genome recombinant adeno-associated virus.

In some embodiments the transgene plasmid is a self-complementary AAV (scAAV) plasmid. Because the conventional ssAAV virus depends on the DNA replication machinery to synthesize the complementary DNA strand, transgene expression may be delayed. To overcome this rate-limiting step, scAAV contains complementary sequences that are capable of spontaneously annealing, upon infection, which eliminates the requirement for host cell DNA synthesis. Methods of generating scAAV plasmids are well known to the person skilled in the art. In some embodiments, the scAAV plasmid comprises a transgene of about 2.4 kb or less.

In some embodiments, the plasmid system can comprise two or more transgene plasmids. The first transgene plasmid may comprise a 3' splice donor and the second with a 5' splice acceptor. When both plasmids are expressed in a cell, they can form concatemers, are spliced together, and the full-length transgene can then be expressed. In other embodiments, a transgene is divided between two transgene plasmids, but with substantial sequence overlap. Co-expression can induce homologous recombination and expression of the full-length transgene.

In some embodiments, the 3' and 5' inverted terminal repeats are independently derived from an ITR sequence of any AAV serotype, including but not limited to, AAV type 1 (e.g., AAV of serotype 1, also referred to as AAV1), AAV type2 (e.g, AAV2), AAV type 3 (e.g, AAV3, including types 3A and 3B, AAV3A and AAV3B), AAV type 4 (e.g, AAV4), AAV type 5 (e.g, AAV5), AAV type 6 (e.g, AAV 6), AAV type 7 (e.g, AAV7), AAV type 8 (e.g, AAV8), AAV type 9 (e.g, AAV9), AAV type 10 (e.g, AAV10), AAV type 11 (e.g, AAV 11), AAV type 12 (e.g, AAV 12), AAV type 13 (e.g, AAV 13), AAV type rh32.33 (e.g, AAVrh32.33), AAV type rh8 (e.g, AAVrh8), AAV type rhlO (e.g, AAVrhlO), AAV type rh74 (e.g, AAVrh74), AAV type hu.68 (e.g, AAVhu.68), avian AAV (e.g, AAAV), bovine AAV (e.g, BAAV), canine AAV, equine AAV, ovine AAV, snake AAV, bearded dragon AAV, AAV2i8, AAV2g9, AAV-LK03, AAV7m8, AAV Anc80, AAV PHP.B, and any other AAV now known or later discovered. In preferred embodiments, the 3' and 5' inverted terminal repeats are derived from J01901.1 AAV2.

In some embodiments, the inverted terminal repeats, preferably the 5' inverted terminal repeats, has a deletion compared to the native inverted terminal repeats. In some embodiments, the transgene plasmid comprising the 5'ITR deletion is a scAAV plasmid.

In some embodiments, the transgene plasmid comprises a cap protein coding sequence.

The transgene plasmid can comprise any suitable promoter. Exemplary suitable promoters can be a chicken β-actin (CBA) promoter, a short CMV early enhancer/chicken β actin (sCAG) promoter, human cytomegalovirus (hCMV) promoter, mouse phosphoglycerate kinase (mPGK) promoter, and human synapsin (hSYN) promoter. In preferred embodiments, the promoter is a CMVie promoter.

In preferred embodiments, the transgene is a reporter gene. In preferred embodiments, the reporter gene can be detected by antibody-based assays. In further preferred embodiments, the reporter gene is a fluorescent molecule. Exemplary fluorescent molecules suitable as reporter gene are GFP, eGFP, mGFP, eYFP, citrine, eCFP, mCFP, Cerulean, dtTomato, and any variants thereof. In some embodiments, the reporter gene is a beta-galactosidase, luciferase or glutathione S-transferase, or any variant thereof.

In some embodiments, the transgene has at most 4.4 kb. In some embodiments, the transgene has at most 2.4 kb.

In some embodiments, the polyadenylation signal sequence is capable of forming a proper poly(A) sequence at the RNA's 3' end. Exemplary polyadenylation signal sequences are an adenovirus L3 poly(A) signal sequence, HSV TK poly(A) signal sequence, hGH poly(A) signal sequence, spA poly(A) signal sequence, rabbit gbpA poly(A) signal sequence, sNRP1 poly(A) signal sequence, bGH poly(A) signal sequence, synthetic poly(A) signal sequence, mouse β-globin poly(A) signal sequence, rabbit β-globin poly(A) signal sequence, H4-based poly(A) signal sequence, and SV40 poly(A) signal sequence. In preferred embodiments, the polyadenylation signal sequence is a SV40 poly(A) signal sequence.

In preferred embodiments, the transgene plasmid comprises the following structure:
5'ITR(flip)-CMVie promotor-transgene-SV40 PolyA-ITR(flop)3'.

In preferred embodiments, the inverted terminal repeats with the flip orientation have the SEQ ID NO: 1 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the inverted terminal repeats with the flop orientation have the SEQ ID NO: 2 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In some embodiments, the transgene plasmid does not contain a woodchuck hepatitis posttranscriptional regulatory element.

In some embodiments, the plasmid system comprises the rep plasmid of the invention which does not comprise a cap protein coding sequence. In some such embodiments, the plasmid system comprises a cap plasmid. The cap plasmid can comprise at least one adeno-associated virus capsid protein coding sequence encoding at least one functional cap protein. The capsid protein coding sequence can encode one or more of VP1, VP2, and VP3, or artificial variants thereof. In preferred embodiments, the capsid protein coding sequence is derived from AAV2.

In preferred embodiments, the VP1 protein comprises or consists of the SEQ ID NO: 12 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the VP2 protein comprises or consists of the SEQ ID NO: 13 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the VP3 protein comprises or consists of the SEQ ID NO: 14 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In particularly preferred embodiments, the plasmid system comprises a transgene plasmid of the invention. In some embodiments, the transgene plasmid is a scAAV transgene plasmid. In some embodiments, the transgene plasmid is a ssAAV transgene plasmid. In some embodiments, the plasmid system can comprise two or more transgene plasmids of the invention.

In some embodiments, the plasmid system is used for rAAV production, preferably transient rAAV production, wherein the rAAV can be used in gene therapy applications.

### The transgene plasmid of the invention

The present invention further provides a transgene plasmid comprising: a CMVie promotor, a transgene, SV40 Poly A, a 5' and a 3' inverted terminal repeats derived from J01901.1 AAV2; wherein the transgene plasmid is selected from one of the following:
(i) conventional single-stranded genome recombinant adeno-associated virus, or
(ii) self-complementary genome recombinant adeno-associated virus.

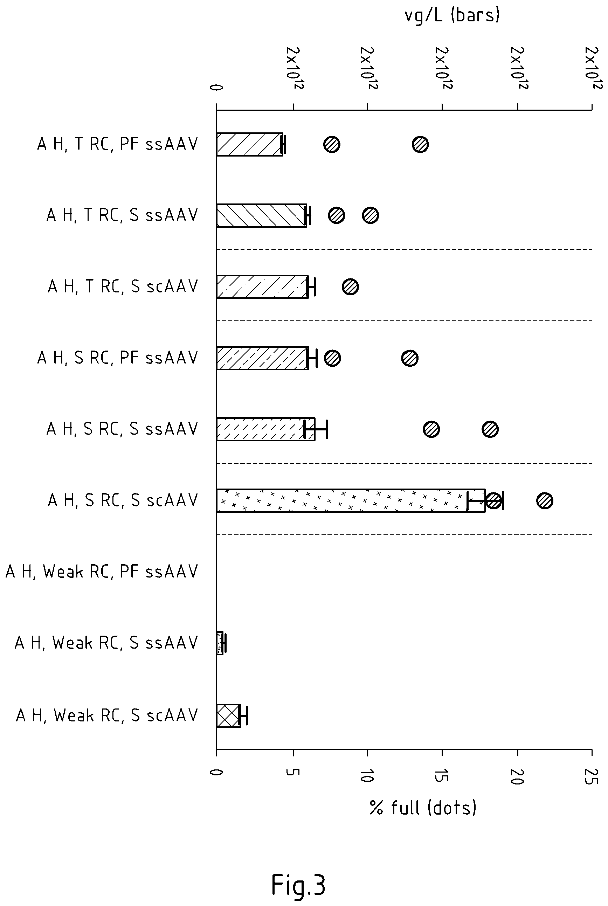

As shown in fig. 3, the transgene plasmid of the invention surprisingly leads to improved concentration of viral genomes, and thus to improved replication for the ssAAV transgene plasmid as well as for the scAAV transgene plasmid. During AAV production, the transgene, i.e., a nucleic acid sequence encoding for a protein of interest, flanked by ITRs, is packaged into an AAV particle and this AAV particle can be used for gene therapy.

In some embodiments, the transgene plasmid is a self-complementary AAV (scAAV) plasmid. A potential downside of AAV can be its single-stranded DNA (ssDNA) genome. Because the virus depends on the DNA replication machinery to synthesize the complementary strand, transgene expression may be delayed. Any ssrAAV genome that does reach the nucleus will still require the synthesis, or recruitment, of a complementary strand in order to achieve gene expression. To overcome this rate-limiting step, scAAV contains complementary sequences that are capable of spontaneously annealing (SA), upon infection, which eliminates the requirement for host cell DNA synthesis. This inter-strand base pairing, or strand annealing, is possible because AAV, unlike many of the autonomous parvoviruses, packages either the plus or minus DNA strand with equal efficiency. The need for dsDNA conversion, either by SA or DNA synthesis, can be circumvented by packaging both strands as a single molecule. This can be achieved by taking advantage of the tendency to produce dimeric inverted repeat genomes during the AAV replication cycle. These dimers can be packaged in the same manner as conventional AAV genomes, and the two halves of the ssDNA molecule can fold and base pair to form a dsDNA molecule of half the length. Although this further restricts the transgene carrying capacity of an already small viral vector, it offers a substantial premium in the efficiency, and speed of onset, of transgene expression because dsDNA conversion is independent of host-cell DNA synthesis and vector concentration. The yield of scAAV genomes can be increased by inhibiting resolution at one terminal repeat. This is normally accomplished by deleting a terminal resolution site sequence from one ITR, such that the Rep protein cannot generate the essential ssDNA nick. The replication complex initiated at the other ITR then copies through the hairpin and back toward the initiating end. Replication proceeds to the end of the template molecule, leaving the mutated ITR in the middle. This dimeric inverted repeat can then undergo normal rounds of replication. Each displaced daughter strand comprises a ssDNA inverted repeat with a ITR at each end and a mutated ITR in the middle. Production and purification of scAAV vector from mutated ITR constructs is the same as conventional ssAAV.

In preferred embodiments, the inverted terminal repeats, preferably the 5' inverted terminal repeats, has a deletion compared to the native inverted terminal repeats. In some embodiments, the transgene plasmid comprising the deletion is a scAAV transgene plasmid.

The transgene plasmid is constructed using known techniques to at least provide operatively linked components in the direction of transcription, control elements including a transcriptional initiation region, the DNA of interest and a transcriptional termination region. The control elements are selected to be functional in a mammalian cell. The resulting construct, which contains the operatively linked components, is flanked (5' and 3') with functional AAV ITR sequences. Termination signals, such as polyadenylation sites, can also be included in the plasmid.

Without wishing to be bound by theory, the ITR is considered to be the only cis elements required for packaging allowing to produce rAAV. Even though the rolling-circle DNA replication mechanism primarily amplifies (i.e., replicates) the transgene expression cassette DNA sequence flanked by the ITRs due to the presence of a D sequence within the ITRs, the plasmid DNA backbone (e.g., origin of replication, antibiotic resistance gene expression cassette, etc...) can also be packaged into the vector capsid, albeit at a lower frequency due to the absence of the flanking D sequence domain. AAV is efficient in packaging a genome size similar to or smaller than the wildtype virus genome (-4.7 kbases). One could discourage the packaging of the plasmid backbone by increasing the size of the backbone to such a degree that it is unfavorable for the backbone to be packaged into the capsid. Enlargement of the backbone can be achieved by additional "stuffer" sequences (i.e., filler component), resulting in a plasmid backbone size larger than the wild-type AAV genome. It is suggested that the presence of an enlarged plasmid backbone can reduce the probability of the rAAV packaging the plasmid backbone into the vector capsid. In some embodiments, the plasmid backbone is enlarged by the use of a stuffer sequence. In certain embodiments, the stuffer sequence is silent in terms of biological activity, in that it is devoid of at least one of enhancers, promoters, splicing regulators, noncoding RNAs, antisense sequences, and/or coding sequences. In certain embodiments, each of enhancers, promoters, splicing regulators, noncoding RNAs, antisense sequences, and coding sequences are absent in the stuffer sequence. In certain embodiments, the stuffer sequence comprises an inert intronic DNA sequence found in the human genome. By utilizing a DNA sequence from the human genome, it is suggested that there will be lower probability that the stuffer sequence may elicit an immune response in case the plasmid becomes packaged into the capsid.

In preferred embodiments, the transgene plasmid comprises the following structure:
5'ITR(flip)-CMVie promotor-transgene-SV40 PolyA-ITR(flop)3'.

In preferred embodiments, the inverted terminal repeats with the flip orientation has the SEQ ID NO: 1 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO

In preferred embodiments, the inverted terminal repeats with the flop orientation has the SEQ ID NO: 2 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the inverted terminal repeats comprises a deletion and has the SEQ ID NO: 4 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

In preferred embodiments, the transgene is a reporter gene. In preferred embodiments, the reporter gene can be detected by antibody-based assays. In further preferred embodiments, the reporter gene is a fluorescent molecule. Exemplary fluorescent molecules suitable as reporter gene are GFP, eGFP, mGFP, eYFP, citrine, eCFP, mCFP, Cerulean, dtTomato, and any variants thereof. In some embodiments, the reporter gene is a beta-galactosidase, luciferase or glutathione S-transferase, or any variant thereof. In some embodiments, the transgene has at most 4.4 kb. In some embodiments, the transgene has at most 2.4 kb. In some preferred embodiments, the reporter gene has a sequence of SEQ ID NO: 17 or preferably at least 85% identity to said SEQ ID NO, more preferably at least 90% identity to said SEQ ID NO, even more preferably at least 95% identity to said SEQ ID NO, most preferably at least 99% identity to said SEQ ID NO.

In preferred embodiments, the transgene plasmid does not contain a woodchuck hepatitis posttranscriptional regulatory element.

In preferred embodiments, the transgene plasmid has a sequence of SEQ ID NO: 3 or preferably at least 85% identity to said SEQ ID NO, more preferably at least 90% identity to said SEQ ID NO, even more preferably at least 95% identity to said SEQ ID NO, most preferably at least 99% identity to said SEQ ID NO.

In preferred embodiments, the transgene plasmid has a sequence of SEQ ID NO: 5 or preferably at least 85% identity to said SEQ ID NO, more preferably at least 90% identity to said SEQ ID NO, even more preferably at least 95% identity to said SEQ ID NO, most preferably at least 99% identity to said SEQ ID NO.

In some embodiments, the transgene plasmid is used for rAAV production, preferably transient rAAV production,wherein the rAAV can be used in gene therapy applications.

In some particularly preferred embodiments, the rep plasmid comprises a minimal element in reverse orientation, wherein the minimal element comprises a sequence shown in SEQ ID NO.: 18, preferably SEQ ID NO.: 6, wherein the sequence is derived from a 3'inverted terminal repeats sequence from J01901.1 AAV2. In some of these embodiments, the rep plasmid is a rep cap plasmid, further comprising a capsid protein coding sequence.

In some particularly preferred embodiments, the transgene plasmid comprises the structure:
5'ITR(flip)-CMVie promotor-transgene-SV40 PolyA-ITR(flop)3',
wherein the transgene is a reporter gene, preferably a fluorescent reporter gene having a sequence shown in SEQ ID NO: 17, and wherein inverted terminal repeats with the flip orientation have a sequence shown in SEQ ID NO.: 1 and the inverted terminal repeats with the flop orientation have a sequence shown in SEQ ID NO.: 2.

### The stable or transient cell expression system and the cell of the invention

The present invention provides a stable or transient cell expression system comprising the plasmid system of the invention and a cell line. In some embodiments, the stable or transient cell expression system comprises the rep plasmid of the invention and the transgene plasmid of the invention. In some embodiments, the stable or transient cell expression system comprises the rep plasmid of the invention. In some embodiments, the stable or transient cell expression system comprises the transgene plasmid of the invention. In some embodiments, the stable or transient cell expression system comprises the plasmid system of the invention. In some embodiments, the stable or transient cell expression system comprises the rep plasmid of the invention and a cap plasmid. In some embodiments, the stable or transient cell expression system comprises the rep plasmid of the invention, the cap plasmid, and a helper plasmid. In some embodiments, the stable or transient cell expression system comprises the rep plasmid of the invention and the helper plasmid. In some embodiments, the stable or transient cell expression system comprises the rep plasmid of the invention and the helper plasmid.

The present invention further provides a cell comprising the rep plasmid of the invention, the plasmid system of the invention, or the transgene plasmid of the invention. In some embodiments, the cell comprises the rep plasmid of the invention and the transgene plasmid of the invention. In some embodiments, the cell comprises the rep plasmid of the invention. In some embodiments, the cell comprises the transgene plasmid of the invention. In some embodiments, the cell comprises the plasmid system of the invention. In some embodiments, the cell comprises the rep plasmid of the invention and a cap plasmid. In some embodiments, the cell comprises the rep plasmid of the invention, the cap plasmid, and a helper plasmid. In some embodiments, the cell comprises the rep plasmid of the invention and the helper plasmid. In some embodiments, the cell comprises the rep plasmid of the invention and the helper plasmid.

In preferred embodiments, the cell is a mammalian cell. The cells can be HEK293 cells, HELA cells or insect cells, or derivatives. Most preferably, the cell is a HEK293 cell.

### The kit of the invention

The present invention provides a kit comprising a stable or or transient cell expression system of the invention, or a cell of the invention, and a cell culture medium.

In some embodiments, the kit comprises media feeds, media additives or transfection reagents or any combination thereof. In some embodiments, the kit comprises a manual.

### The method of producing recombinant adeno-associated viral vectors of the invention

The present invention provides a method of producing recombinant adeno-associated viral vectors comprising:
(i) transfecting cells with the rep plasmid of the invention, the plasmid system of the invention, or the transgene plasmid of the invention;
(ii) culturing the transfected cells to produce said adeno-associated viral vectors; and
(iii) isolating said recombinant adeno-associated viral vectors.

The cells can be HEK293 cells, HELA cells or insect cells, or derivatives. In preferred embodiments, the cells are mammalian cells, preferably HEK293 cells.

### SEQUENCE LISTING

This application contains a Sequence Listing which has been submitted electronically and is hereby incorporated by reference in its entirety. Said Sequence Listing file is named 230267EP_Sequence Listing.XML and 29,000 Bytes in size.
SEQ ID NO: 1 is an exemplary polynucleotide sequence of a 5' ITR sequence suitable for use in the transgene plasmid of the disclosure.
SEQ ID NO: 2 is an exemplary polynucleotide sequence of a 3' ITR sequence suitable for use in the transgene plasmid of the disclosure.
SEQ ID NO: 3 is an exemplary polynucleotide sequence of a ssrAAV transgene plasmid of the disclosure.
SEQ ID NO: 4 is an exemplary polynucleotide sequence of a 5' ITR sequence comprising a deletion and being suitable for use in the transgene plasmid of the invention, in particular the scrAAV transgene plasmid of the disclosure.
SEQ ID NO: 5 is an exemplary polynucleotide sequence of a scrAAV transgene plasmid of the disclosure.
SEQ ID NO: 6 is an exemplary polynucleotide sequence of a minimal element suitable for use in the rep plasmid of the disclosure.
SEQ ID NO: 7 is an exemplary polynucleotide sequence of a rep transgene plasmid of the disclosure.
SEQ ID NOs: 8, 9, 10, and 11 are exemplary amino acid sequences of the rep78, rep68, rep52, and rep 40 proteins, respectively.
SEQ ID NOs: 12, 13, and 14 are exemplary amino acid sequences of the VP1, VP2, and VP3 proteins, respectively.
SEQ ID NOs: 15 and 16 are exemplary amino acid sequences of the AAP and X proteins, respectively.
SEQ ID NO: 17 is an exemplary amino acid sequence of an eGFP fluorescent reporter for use in the transgene plasmid of the disclosure.
SEQ ID NO: 18 is an exemplary polynucleotide sequence comprising or consisting of a rep-binding element (RBE).

### EXAMPLES

### Example 1:

AAVMax^{©} cells (Thermo) were grown in an Ambr15 for 4 days at DO of 40, rpm of 630, pH range of 7.2 - 7.0 in HEK VIP NB medium (Sartorius Xell) with cell densities maintained between 0.5 and 4 million viable cells/mL. On day 5 the cells were transfected at a density of 3.5-4 million viable cells/mL with 1 ug of plasmid per million viable cells and 1.8 ug PEIPro (PolyPlus) million viable cells Plasmid molar ratios were 1:1 (2-plasmid system) or 1:1:1 (3-plasmid system) and transfection mix volume was 15% v/v final volume. Twenty-four hours post transfection cells were fed by addition of 10% v/v FS (Sartorius Xell). Seventy-two hours post transfection cell suspension was lysed and Benzonase digested and ddPCR performed as well as capsid ELISA (Progen).

As shown in fig. 1, PlasmidFactory^{©} AAV2-derived plasmids comprising rep, cap, and helper genes (PF HRC) has been combined with Plasmidfactory^{©} ssAAV CMV-GFP transgene plasmids (PF ssAAV) to produce ssrAAV capsids (pAAV-ssGFP) using the Two-Plasmid-System. Similarly, PlasmidFactory^{©} AAV2-derived plasmids comprising rep, cap, and helper genes (PF HRC) has been combined with the transgene plasmids of the invention (S ssAAV) to produce ssAAV capsids (optimized pAAV-ssGFP) using the Two-Plasmid-System. The measured genomic titer is surprisingly increased in the Two-Plasmid-System comprising the improved transgene plasmid. As the same time, the percentage of full capsids were also increased.

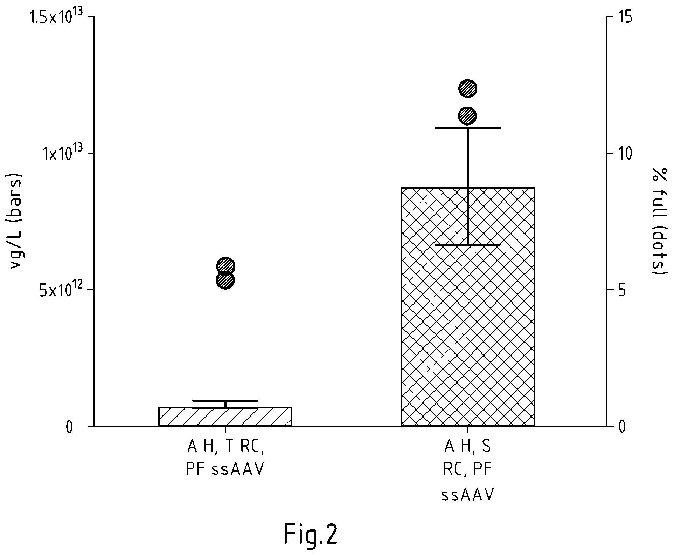

As shown in fig. 2, Takara^{©} AAV2-derived plasmids comprising rep and cap genes (T RC) were combined with Aldevron^{©} pHelper plasmids (A H) and Plasmidfactory^{©} ssrAAV GFP transgene plasmids (PF ssAAV) to produce ssrAAV capsids (pAAV RC2) using the Three-Plasmid-System. Similarly, rep plasmids comprising cap coding sequence and the minimal element comprising a rep-binding element downstream of the polyadenylation signal sequence (S RC) has been combined with Plasmidfactory^{©} ssrAAV GFP transgene plasmids (PF ssAAV) and Aldevron^{©} pHelper plasmids (A H) a to produce ssrAAV capsids (optimized pAAV RC2) using the Three -Plasmid-System. The measured genomic titer and full capsids are substantially improved in the Three-Plasmid-System comprising the rep plasmid comprising the minimal element downstream the polyadenylation signal.

This demonstrates that the transgene plasmid and rep plasmid of the invention can be advantageously used in an rAAV production process as the replication and packaging in capsids are more efficient compared to conventional plasmids.

### Example 2:

AA VMax^{©} cells (Thermo) or HEK293 clones C1, C2, C3 or genetically modified HEK293 (H-GMO) cells were grown in shakeflasks in 80% relative humidity, 37°C, 5 % CO₂ and 185 rpm in TF medium (Sartorius Xell) with cell densities maintained between 0.5 and 4 million viable cells/mL. Transfection was performed at a density of 3.5-4 million viable cells/mL with 1 ug of plasmid per million viable cells and 1.8 ug PEIPro (PolyPlus) per million viable cells. Plasmid molar ratios were 1:1:1 and transfection mix volume was 15% v/v final volume. Twenty-four post transfection cells were fed by addition of 10% v/v FS (Sartorius Xell). Seventy-two hours post transfection cell suspension was lysed and Benzonase digested and ddPCR performed as well as capsid ELISA (Progen) and in some cases GFP based potency test (transduction of suspension HEK cells).

As shown in fig 3, a Three-Plasmid System were used with Thermo AA VMax^{©} cells in shakeflask.

The following combinations have been tested:
- Takara AAV2 Rep Cap (RC) plasmid (T RC) with PlasmidFactory GFP ssAAV (PF ssAAV)
- Takara AAV2 RC plasmid (T RC) with Sartorius GFP ssAAV (S ssAAV)
- Takara AAV2 RC plasmid (T RC) with Sartorius GFP scAAV (S scAAV)
- Sartorius RC (S RC) with PlasmidFactory GFP ssAAV (PF ssAAV)
- Sartorius RC (S RC) with Sartorius GFP ssAAV (S ssAAV)
- Sartorius RC (S RC) with Sartorius GFP scAAV (S scAAV)
- control AAV2 Rep Cap plasmid with very weak rep expression (Weak RC) with PF ssAAV
- weak RC with Sartorius GFP ssAAV (S ssAAV)
- weak RC with Sartorius GFP scAAV (S scAAV)

All of the above combinations have been tested with Aldevron^{©} Helper pALD-X80 (A H). The tested "Sartorius GFP ssAAV" and "Sartorius GFP scAAV" refer to the ssAAV transgene plasmids and scAAV transgene plasmids provided by the present disclosure, wherein the transgene is a GFP reporter gene.

These examples confirm that the addition of the transgene plasmid of the invention advantageously results in increased genomic titer compared to the prior art PF ssAAV transgene plasmid. Even if rep is weakly expressed, leading to no detectable genomic titer with the conventional plasmid, an increased titer can be obtained by use of the transgene plasmid provided by the present invention. In addition, an improved titer is obtained when combing the rep plasmid (Sartorius RC) with the conventional transgene plasmid PF ssAAV. A further improved titer is provided when combining the rep plasmid (Sartorius RC) with any one of the S ssAAV or S scAAV transgene plasmids. Most strikingly, a synergistic effect in genomic titer and full capsids is achieved for the rep plasmid and scAAV transgene (S RC and S scAAV) plasmid system.

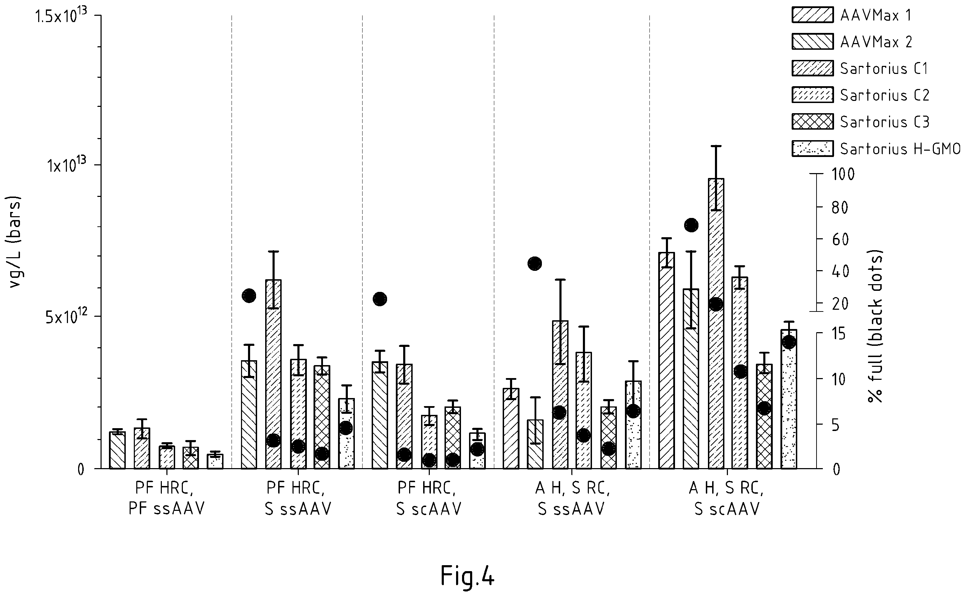

In fig 4, a summary is shown for various HEK cells (pools or clones) tested with Two- and Three-Plasmid-System in shakeflask.

The following combinations have been tested in a Two-Plasmid-System:
- PlasmidFactory AAV2-derived rep/cap/helper plasmids (PF HRC) with PlasmidFactory single-stranded Gene-Of-Interest (GOI) (PF ssAAV)
- PF HRC with Sartorius ssGOI (S ssAAV)
- PF HRC with Sartorius scGOI (S scAAV)

The following combinations have been tested in a Three-Plasmid-System:
- Aldevron Helper pALD-X80 (A H) with Sartorius RC (S RC) and Sartorius ssGOI (S ssAAV)
- Aldevron H (A H) with Sartorius RC (S RC) and Sartorius scGOI (S scAAV)

The tested "Sartorius ssGOI" and "Sartorius scGOI" refers to the ssAAV transgene plasmids and scAAV transgene plasmids disclosed herein, wherein the transgene is a GFP reporter gene. The "Sartorius RC" refers to the rep plasmid comprising a minimal element comprising a rep-binding element downstream of the polyadenylation signal sequence, wherein the rep-binding element is in reverse orientation. This rep plasmid further comprises a cap coding sequence.

It is demonstrated that the scAAV and ssAAV transgene plasmids of the invention advantageously result in increased genomic titer even when used in a plasmid system comprising conventional rep/cap plasmids. A combination of the rep plasmid (Sartorius RC, "S RC") with the any one of the ssAAV or scAAV transgene plasmids further leads to a higher titer, with a synergistic effect in genomic titer being again obtained for the rep plasmid and scAAV transgene plasmid system.

### Example 3:

Sartorius HEK293 clone 3 was grown in shakeflasks in 80% relative humidity, 37°C, 5 % CO₂ and 185 rpm in TF medium (Sartorius Xell) with cell densities maintained between 0.5 and 4 million viable cells/mL. Transfection was performed at a density of 3.5-4 million viable cells/mL with 1 ug of plasmid per million viable cells and 1 ug PEIMax (Polysciences) per million viable cells. Plasmid molar ratios were 1:1:1 and transfection mix volume was 10% v/v final volume. Seventy-two hours post transfection cell suspension was lysed and Benzonase digested and ddPCR performed.

The following combinations have been tested:
- Two-Plasmid-System: PlasmidFactory AAV2-derived rep/cap/helper plasmids (PF HRC) with PlasmidFactory single-stranded Gene-Of-Interest (PF ssAAV)
- Three-Plasmid-System: Aldevron H (A H), Sartorius RC (S RC), Sartorius ssAAV (S ssAAC)

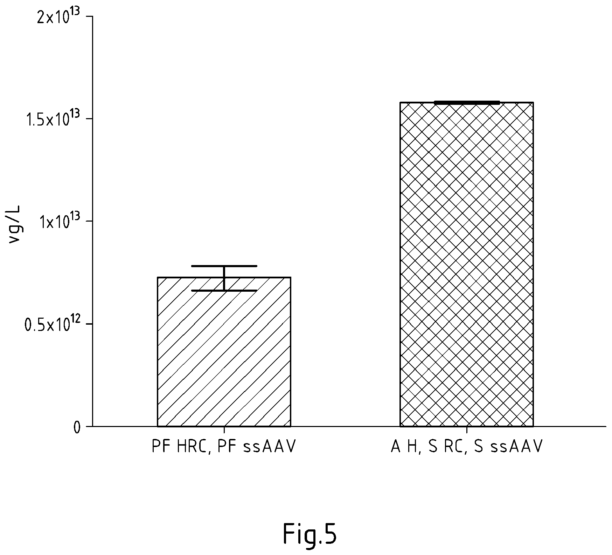

As shown in fig. 5, the combination of the rep plasmid (Sartorius RC, "S RC") with the ssAAV transgene plasmids leads to a higher titer. These examples also confirm that the addition of the rep plasmid and transgene plasmid of the invention advantageously results in increased genomic titer compared to the prior art plasmid system.

### Example 4:

Thermo EXPI293F IC were grown in shakeflasks in 80% relative humidity, 37°C, 8 % CO₂ and 130 rpm in Freestyle 293 Expression Medium (Thermo) with cell densities maintained between 0.2 and 3 million viable cells/mL. Transfection was performed 2 days after seeding at a density of 1.3 - 1.5 million viable cells/mL with 0.55 ug of plasmid per million viable cells and 0.55 ul FectoVir (PolyPlus) per million viable cells. Plasmid molar ratios were 4.5:1 (ssAAV : HRC) in Freestyle 293 Expression Medium and transfection mix was 5% v/v of final culture volume. Seventy-two hours post transfection cell suspension was lysed and Denarase digested and ddPCR performed as well as capsid ELISA (Progen).

The following combinations have been tested in two replications (repl. 1 and 2):
- PlasmidFactory rep/cap/helper plasmids (PF HRC) derived from AAV8 isolated from the ape species with PlasmidFactory single-stranded Gene-Of-Interest (PF ssAAV)
- PlasmidFactory rep/cap/helper plasmids (PF HRC) derived from AAV8 isolated from the ape species with Sartorius ssAAV (S ssAAC)

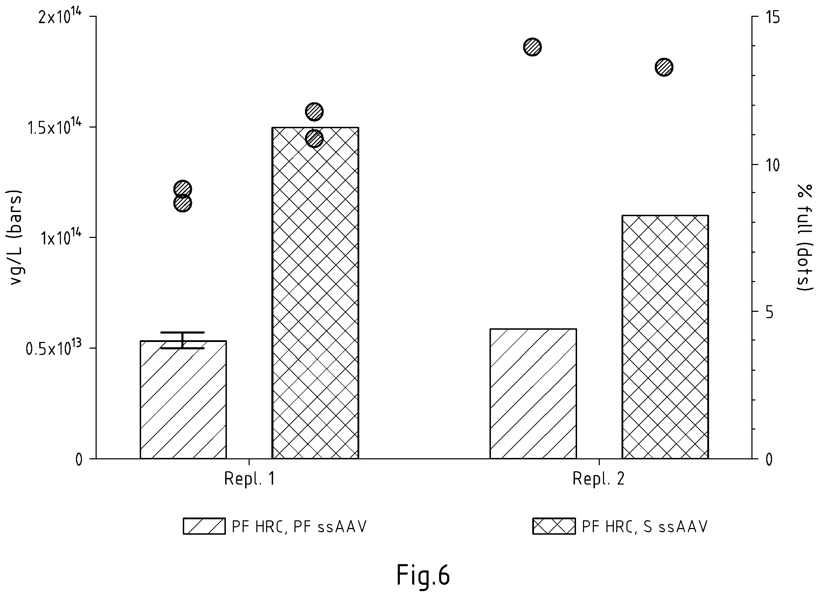

As shown in fig. 6, the transgene plasmid disclosed herein advantageously results in increased genomic titer compared to the prior art ssAAV transgene plasmid.

These experiments confirm the proper rAAV production by the rep plasmid of the invention and by the transgene plasmid of the invention. As demonstrated above, the rep plasmid and transgene plasmid surprisingly lead to a higher titer and improved rAAV production. The improvement in genomic titer achieved by using the plasmids according to the present disclosure were reproducibly obtained using different transfection reagents (PEIpro, PEIMax, FectoVir), showing the robustness of the plasmids.

It should be understood from the foregoing that, while particular implementations have been illustrated and described, various modifications can be made thereto and are contemplated herein. It is also not intended that the invention be limited by the specific examples provided within the specification. While the invention has been described with reference to the aforementioned specification, the descriptions and illustrations of the preferable embodiments herein are not meant to be construed in a limiting sense. Furthermore, it shall be understood that all aspects of the invention are not limited to the specific depictions, configurations or relative proportions set forth herein which depend upon a variety of conditions and variables. Various modifications in form and detail of the embodiments of the invention will be apparent to a person skilled in the art. It is therefore contemplated that the invention shall also cover any such modifications, variations and equivalents. It is intended that the following claims define the scope of the invention and that methods and structures within the scope of these claims and their equivalents be covered thereby.

### BRIEF DESCRIPTION OF THE DRAWING

Fig 1. depicts the measured concentration of viral genomes [vg/L (bars)] and full capsids [% full (dots)] when using a Two-Plasmid-System of conventional plasmids (PF HRC, PF ssAAV) compared to a Two-Plasmid-System comprising the transgene plasmid of the invention (PF HRC, S ssAAV).
Fig 2. depicts the measured concentration of viral genomes [vg/L (bars)] and full capsids [% full (dots)] when using a Three-Plasmid-System of conventional plasmids (A H, T RC, PF ssAAV) compared to a Three-Plasmid-System comprising the rep plasmid of the invention (A H, S RC, PF ssAAV).
Fig 3. depicts the measured concentration of viral genomes [vg/L (bars)] and full capsids [% full (dots)] when using a Three-Plasmid-System of various combinations of conventional rep-cap plasmids (T RC or Weak RC), conventional transgene plasmids (PF ssAAV), the rep plasmid of the invention (S RC) and the transgene plasmid of the invention (S ssAAV, S scAAV).
Fig 4. depicts the measured concentration of viral genomes [vg/L] and full capsids [% full (dots)] when using a Two- or Three-Plasmid-System of various combinations of conventional rep-cap plasmids (PF HRC), conventional transgene plasmids (PF ssAAV), the rep plasmid of the invention (S RC) and the transgene plasmids of the invention (S ssAAV, S scAAV).
Fig. 5 depicts the measured concentration of viral genomes [vg/L] when using a Two-Plasmid-System of a conventional rep-cap plasmids (PF HRC), conventional transgene plasmids (PF ssAAV) and a Three-Plasmid-System comprising the rep plasmid of the invention (S RC) and the transgene plasmid of the invention (S ssAAV).
Fig. 6 depicts the measured concentration of viral genomes [vg/L] and full capsids [% full (dots)] when using a Two-Plasmid-System of a conventional rep-cap plasmids (PF HRC) and either conventional transgene plasmids (PF ssAAV) or the transgene plasmid of the invention (S ssAAV).

## Claims

1. A rep plasmid comprising:
(i) at least one adeno-associated virus replication protein coding sequence encoding at least one functional rep protein;
(ii) at least one polyadenylation signal sequence downstream the adeno-associated virus replication protein coding sequence; and
(iii) a minimal element comprising one or more recombinant rep-binding elements downstream the polyadenylation signal sequence.

2. The rep plasmid of claim 1, wherein the one or more rep-binding elements function as binding site for rep68 and/or rep78.

3. The rep plasmid of claim 1 or 2, wherein the rep-binding element comprises the SEQ ID NO: 18 or a sequence having at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

4. The rep plasmid of any of claims 1 to 3, wherein the rep-binding element is derived from an inverted terminal repeats sequence, preferably derived from a 3'inverted terminal repeats sequence derived from J01901.1 AAV2.

5. The rep plasmid of any of claims 1 to 4, wherein the minimal element comprises the SEQ ID NO: 6 or a sequence having at least 60%, at least 70%, at least 80% identity to said SEQ ID NO, preferably a sequence having at least 85% identity to said SEQ ID NO, more preferably a sequence having at least 90% identity to said SEQ ID NO, even more preferably a sequence having at least 95% identity to said SEQ ID NO, most preferably a sequence having at least 99% identity to said SEQ ID NO.

6. A plasmid system for producing an adeno-associated virus particle comprising:
(i) a transgene plasmid; and
(ii) the rep plasmid of any of claims 1 to 5.

7. The plasmid system of claim 6, wherein the system further comprises a helper plasmid, wherein the helper plasmid preferably comprises one or more coding sequences encoding for E2A, E4orf6, or E4orf7 or any combination thereof.

8. The plasmid system of claim 6 or 7, wherein the transgene plasmid comprises a promotor, a transgene, a polyadenylation signal sequence, 5' and 3' inverted terminal repeats,
wherein the transgene plasmid is selected from one of the following:
(i) conventional single-stranded genome recombinant adeno-associated virus, or
(ii) self-complementary genome recombinant adeno-associated virus.

9. The plasmid system of claim 8, wherein the 3' and 5' inverted terminal repeats are derived from J01901.1 AAV2, and/or wherein at least one inverted terminal repeats, preferably the 5' inverted terminal repeats, has a deletion compared to the native inverted terminal repeats.

10. A transgene plasmid comprising:
a CMVie promotor, a transgene, SV40 Poly A, a 5' and a 3' inverted terminal repeats derived from J01901.1 AAV2;
wherein the transgene plasmid is selected from one of the following:
(i) conventional single-stranded genome recombinant adeno-associated virus, or
(ii) self-complementary genome recombinant adeno-associated virus.

11. The transgene plasmid of claim 10, wherein the transgene plasmid comprises the following structure:
5'ITR(flip)-CMVie promotor-transgene-SV40 PolyA-ITR(flop)3'.

12. A stable or transient cell expression system comprising the plasmid system of any one of claims 6 to 9 and a cell line.

13. A cell comprising the rep plasmid of any of claims 1 to 5, the plasmid system of any one of claims 6 to 9, or the transgene plasmid of claim 10 or 11.

14. A kit comprising a stable or or transient cell expression system of claim 12, or a cell of claim 13, and a cell culture medium.

15. A method of producing recombinant adeno-associated viral vectors comprising:
(i) transfecting cells with the rep plasmid of any of claims 1 to 5, the plasmid system of any one of claims 6 to 9, or the transgene plasmid of claim 10 or 11;
(ii) culturing the transfected cells to produce said adeno-associated viral vectors; and
(iii) isolating said recombinant adeno-associated viral vectors.
